# EUROPEAN PATENT SPECIFICATION

(11) **EP 2 090 310 B1**
(45) Date of publication and mention of the grant of the patent: **10.07.2013**
(21) Application number: 07849959.7
(22) Date of filing: 30.11.2007
(51) Int. Cl.: A61K 31/445, A61K 9/70, A61K 47/02, A61K 47/14, A61K 47/32, A61P 25/28, A61K 47/22, A61K 47/20

(54) **METHOD FOR PREVENTION OF COLORATION IN DONEPEZIL-CONTAINING SKIN ADHESIVE PREPARATION, AND METHOD FOR REDUCING THE PRODUCTION OF DONEPEZIL ANALOGUE IN DONEPEZIL-CONTAINING SKIN ADHESIVE PREPARATION**
VERFAHREN ZUR PRÄVENTION VON VERFÄRBUNG IN EINER DONEPEZILHALTIGEN HAUTKLEBEZUBEREITUNG UND VERFAHREN ZUR REDUZIERUNG DER PRODUKTION VON DONEPEZIL-ANALOGA IN EINER DONEPEZILHALTIGEN HAUTKLEBEZUBEREITUNG
PROCEDES DESTINES A PREVENIR LA COLORATION DANS UNE PREPARATION D'ADHESIF CUTANE CONTENANT DU DONEPEZIL, PROCEDES DE REDUCTION DE LA PRODUCTION D'ANALOGUE DE DONEPEZIL DANS UNE PREPARATION D'ADHESIF CUTANE

(30) Priority: 01.12.2006 JP 2006325104; 01.12.2006 US 861965 P; 07.06.2007 JP 2007152016; 07.06.2007 JP 2007152047
(43) Date of publication of application: 19.08.2009
(73) Proprietor: Nitto Denko Corporation, Ibaraki-shi, Osaka 567-8680 (JP); Eisai R&D Management Co., Ltd., Tokyo 112-8088 (JP)
(72) Inventor: NISHI, Sumiyo, Ibaraki-shi Osaka 567-8680 (JP); HANATANI, Akinori, Ibaraki-shi Osaka 567-8680 (JP); SEKIYA, Junichi, Ibaraki-shi Osaka 567-8680 (JP); TERASHI, Sachiko, Ibaraki-shi Osaka 567-8680 (JP); AKEMI, Hitoshi, Ibaraki-shi Osaka 567-8680 (JP); WASHIRO, Satoko, Ibaraki-shi Osaka 567-8680 (JP)
(74) Representative: HOFFMANN EITLE
(86) International application number: PCT/JP2007/073253
(87) International publication number: WO 2008/066184

(56) References cited:
- EP-A1- 1 086 706
- EP-A1- 1 437 130
- EP-A1- 1 541 177
- WO-A1-2006/070930
- WO-A1-2006/082728
- JP-A- 2000 136 134
- JP-A- 2007 217 328
- MORIEARTY P L ET AL: "Transdermal patch delivery of acetylcholinesterase inhibitors", METHODS AND FINDINGS IN EXPERIMENTAL AND CLINICAL PHARMACOLOGY, PROUS, BARCELONA, ES, vol. 15, no. 6, 1 January 1993 (1993-01-01), pages 407-412, XP009120198, ISSN: 0379-0355

## Description

### [Field of the Invention]

The present invention relates to a method for suppressing coloring of an adhesive preparation containing donepezil and a method for reducing the amounts of donepezil-related substances formed.

### [Background of the Invention]

The basic drug donepezil has acetylcholine esterase inhibitory action, and is used as an anti-Alzheimer's dementia drug. Alzheimer's dementia patients are usually elderly, and elderly patients often have difficulty swallowing oral preparations. In addition, it may also be difficult in some cases to administer oral preparations to patients with advanced symptoms of Alzheimer's dementia. In these cases, percutaneous parenteral administration of donepezil is useful.

Adhesive preparations containing donepezil for percutaneous parenteral administration of donepezil are known, and are described, for example, Japanese Patent Application Laid-open No. H11-315016 (Patent Document 1), WO 2003/032960 Pamphlet (Patent Document 2), and WO 2006/082728 Pamphlet (Patent Document 3) and the like.

However, a problem encountered by the inventors of the present application during the course of research is that the effective content of donepezil in the preparation significantly declines over time after a donepezil-containing adhesive preparation is prepared. Namely, countermeasures need to be taken to prevent production of donepezil-related substances in the adhesive preparation (pressure-sensitive adhesive layer). However, in an adhesive preparation for parenteral administration, since the environment of the drug present therein is much different from that in an orally administered preparation, and a preparation in sheet form is especially susceptible to the effects of the surrounding environment (oxygen), even if an antioxidant (stabilizer) commonly used in oral preparations is blended with, the donepezil will not necessarily be stabilized, while on the contrary the amounts of donepezil-related substances produced may increase.

Although Patent Documents 2 and 3 describe that antioxidants such as tocopherol and ester derivatives thereof, ascorbic acid, ascorbyl stearate, nordihydroguaiaretic acid, dibutylhydroxytoluene (BHT), butylhydroxyanisole and the like can be used in the pressure-sensitive adhesive layer as necessary, these do not describe any examples of preparations in which antioxidants were actually blended or adequately verify the effectiveness of the antioxidants in the preparations, thus stabilization of donepezil with antioxidants has not been found.

Japanese Patent Application Laid-open No. 2000-136134 (Patent Document 4) describes that increases in donepezil-related substances can be inhibited by adding antioxidants such as sodium hydrogen sulfite, sodium sulfite, sodium pyrosulfite (sodium metabisulfite), cysteine, citric acid, edetate disodium (disodium ethylenediaminetetraacetate), ascorbic acid and erythorbic acid (isoascorbic acid) and the like to an orally administered composition containing donepezil. However, this document proposes a liquid, syrup or other orally administered preparation, and does not suggest application of antioxidants to adhesive preparations.

On the other hand, pharmaceuticals tend to be preferred to have coloring that does not inflict an unpleasant sensation such as seeming artificial for reasons such as imparting a sense of reassurance and cleanliness, and since adhesive preparations in particular are often adhered directly to the skin by a patient, a naturally colored appearance such as a white color is preferred. However, a method has not been found for realizing an appearance of natural coloring by suppressing coloring of adhesive preparations containing donepezil.

For example, Japanese Patent No. 3124069 (Patent Document 5), Japanese Patent Application Laid-open No. H11-047233 (Patent Document 6), Japanese Translation of PCT Application No. 2006-523637 of (Patent Document 7) and Japanese Translation of PCT Application No. 2003-530422 (Patent Document 8) disclose technologies for suppressing discoloration in an adhesive preparation during long-term storage. However, these documents do not teach a method for realizing a naturally colored appearance of the preparation, nor do they teach a method for realizing a naturally colored appearance of an adhesive preparation containing donepezil.

Patent Document 1: Japanese Patent Application Laid-open No. H11-315016
Patent Document 2: WO 2003/032960 Pamphlet
Patent Document 3: WO 2006/082728 Pamphlet
Patent Document 4: Japanese Patent Application Laid-open No. 2000-136134
Patent Document 5: Japanese Patent No. 3124069
Patent Document 6: Japanese Patent Application Laid-open No. H11-047233
Patent Document 7: Japanese Translation of PCT Application No. 2006-523637
Patent Document 8: Japanese Translation of PCT Application No. 2003-530422

### [Disclosure of the Invention]

### [Problems To Be Solved by the Invention]

Under these circumstances, an object of the present invention is to realize a naturally colored appearance of a preparation by suppressing coloring of an adhesive preparation containing donepezil, as well as to reduce the amounts of donepezil-related substances formed.

### [Means for Solving the Problems]

As a result of exhaustive research, the inventors of the present invention found that a naturally colored appearance can be realized by suppressing coloring of a donepezil-containing adhesive preparation by adding two specific species of additives, and that the amounts of specific donepezil-related substances formed, which are found in comparatively large amounts in donepezil-containing adhesive preparations, can be effectively reduced, while also unexpectedly finding that the total amounts of related substances formed is also reduced, thereby leading to completion of the present invention.

Namely, the present invention is as follows:
(1) a method for suppressing coloring of an adhesive preparation having a support and a pressure-sensitive adhesive layer, the pressure-sensitive adhesive layer containing a pressure-sensitive adhesive and donepezil, the method comprising:
   a step of including ascorbic acid, a metal salt or an ester thereof and metal metabisulfite salt in the pressure-sensitive adhesive layer;
(2) the method described in (1) above, which comprises:
   a step of preparing a mixture comprising the pressure-sensitive adhesive, the donepezil, the ascorbic acid, a metal salt or an ester thereof, and the metal metabisulfite salt; and
   a step of film-forming the pressure-sensitive adhesive layer by applying the mixture to a surface of the support;
(3) the method described in (1) or (2) above, comprising:
   blending 0.02 to 1.5 parts by weight of the ascorbic acid, a metal salt or an ester thereof to 1.0 part by weight of the metal metabisulfite salt;
(4) a method for suppressing coloring of an adhesive preparation having a support and a pressure-sensitive adhesive layer, the pressure-sensitive adhesive layer containing a pressure-sensitive adhesive and donepezil, and reducing the amounts of donepezil-related substances formed, the method comprising:
   a step of including ascorbic acid, a metal salt or an ester thereof and metal metabisulfite salt in the pressure-sensitive adhesive layer;
(5) the method described in (4) above, which comprises:
   a step of preparing a mixture comprising the pressure-sensitive adhesive, the donepezil, the ascorbic acid, a metal salt or an ester thereof, and the metal metabisulfite salt, and
   a step of film-forming the pressure-sensitive adhesive layer by applying the mixture to a surface of the support;
(6) the method described in (4) or (5) above, comprising:
   blending 0.02 to 1.5 parts by weight of the ascorbic acid, a metal salt or an ester thereof to 1.0 part by weight of the metal metabisulfite salt;
(7) an adhesive preparation having a support and a pressure-sensitive adhesive layer and comprising an adhesive and donepezil in the pressure-sensitive adhesive layer; wherein, ascorbic acid, a metal salt or an ester thereof and metal metabisulfite salt are comprised in the pressure-sensitive adhesive layer;
(8) the adhesive preparation described in (7) above, wherein the pressure-sensitive adhesive layer comprises the ascorbic acid, a metal salt or an ester thereof at a proportion of 0.02 to 1.5 parts by weight to 1.0 part by weight of the metal metabisulfite salt.
(9) an adhesive preparation having a support and a pressure-sensitive adhesive layer and comprising a pressure-sensitive adhesive and donepezil in the pressure-sensitive adhesive layer, wherein
   the pressure-sensitive adhesive layer is obtained by film-forming of a mixture comprising a pressure-sensitive adhesive, donepezil, ascorbic acid, a metal salt or an ester thereof, and metal metabisulfite salt; and
(10) the adhesive preparation described in (9) above, wherein the mixture comprises the ascorbic acid, a metal salt or an ester thereof at a proportion of 0.02 to 1.5 parts by weight to 1.0 part by weight of the metal metabisulfite salt.

### [Advantageous Effects of the Invention]

According to the present invention, since a naturally colored appearance such as a white color is realized by suppressing coloring of a donepezil-containing adhesive preparation by using combinations of two specific species of additives, a donepezil-containing adhesive preparation can be realized having an appealing appearance.
In addition, the amounts of a specific donepezil-related substance formed in a preparation can be reliably reduced by using combinations of two specific species of additives, a highly safe and stable donepezil-containing adhesive preparation can be realized. Moreover, since not only the amounts of a specific donepezil-related substance formed, but also the total amounts of donepezil-related substances formed can be reduced, an extremely highly safe and stable donepezil-containing adhesive preparation can be achieved.
Moreover, by combining the use of two species of specific additives, the amounts of related substances formed can be inhibited even more (namely, inhibited synergistically) in comparison with the arithmetic mean value of the formed amounts of related substances obtained with each of additives independently. Thus, a highly safe and stable donepezil-containing adhesive preparation can be efficiently obtained without the need to use a large amount of additives.

### [Best Mode for Carrying Out the Invention]

The following provides an explanation of the present invention in accordance with preferred embodiments thereof
The method for suppressing coloring of an adhesive preparation of the present invention is that making donepezil, a pressure-sensitive adhesive and two specific species of additives include in a pressure-sensitive adhesive layer in an adhesive preparation comprising a support and a pressure-sensitive adhesive layer formed on at least one side of the support.

Here, the term "donepezil" encompasses not only (±)-2-[(1-benzylpiperidin-4-yl)methyl]-5,6-dimethoxyindan-1-one (free form), but also pharmacologically acceptable salts and esters thereof.

In the present invention, the donepezil may be either donepezil (free form) or any of pharmaceutically acceptable salts or esters thereof. The pressure-sensitive adhesive layer preferably contains donepezil (free form) from the viewpoint of percutaneous absorbability.

The adhesive preparation of the present invention can be used as an anti-Alzheimer's dementia drug. In addition, other possible applications include use against cerebrovascular dementia, prevention of migraine headaches and the like.

In the adhesive preparation of the present invention, the proportion of donepezil in the pressure-sensitive adhesive layer is preferably 1 to 30 wt% and more preferably 3 to 20 wt% based on the total weight of the pressure-sensitive adhesive layer. If the proportion is less than 1 wt%, an content effective for treatment cannot be expected to be released, while if the proportion exceeds 30 wt%, limitations arise on therapeutic effects while also resulting in the risk of being economically disadvantageous.

The two specific species of additives used in the present invention are ascorbic acid, metal salts or esters thereof (preferably a sodium salt or palmitic acid ester) and metal metabisulfite salt (preferably a sodium salt). As a result of blending these two species of additives, coloring of a donepezil-containing adhesive preparation can be suppressed and a preparation can be made to have a naturally colored appearance. In addition, since these two species of additives also function as stabilizers as will be described later, the amounts of donepezil-related substances formed in the preparation can be reduced.

In the above-mentioned additives, examples of the metal salts include sodium salts, potassium salts, calcium salts and magnesium salts. Examples of the esters include palmitic acid esters, stearic acid esters and myristic acid esters.

Although there are no particular limitations on the ascorbic acid, metal salts or esters thereof and the metal metabisulfite salt, preferably 0.02 to 1.5 parts by weight, more preferably 0.04 to 1.0 part by weight and most preferably 0.04 to 0.2 parts by weight of the ascorbic acid, metal salts or esters thereof are blended based on 1.0 part by weight of the metal metabisulfite salt. Suppression of coloring may be inadequate if the amount of ascorbic acid, metal salts or esters thereof blended is less than 0.02 parts by weight or greater than 1.5 parts by weight.

Although the content of ascorbic acid, metal salts or esters thereof in the adhesive preparation can be suitably set and there are no particular limitations thereon, the content in terms of the solid content thereof is preferably 0.01 to 0.75 wt%, more preferably 0.02 to 0.5 wt%, and most preferably 0.02 to 0.10 wt% based on the total weight of the pressure-sensitive adhesive layer. In addition, although the content of the metal metabisulfite salt in the pressure-sensitive adhesive layer can be suitably set and there are no particular limitations thereon, the content in terms of the solid content thereof is preferably 0.1 to 1.0 wt% and more preferably 0.2 to 0.8 wt% based on the total weight of the pressure-sensitive adhesive layer.

A stabilizer may be blended in the pressure-sensitive adhesive layer in the adhesive preparation of the present invention. Here, the term "stabilizer" means a compound that has the action of being able to inhibit formation of donepezil-related substances (or reducing the amounts of such related substances formed) in a pressure-sensitive adhesive layer containing donepezil and in a mixture of materials used to form the pressure-sensitive adhesive layer. Specific examples of the stabilizer include ascorbic acid, metal salts or esters thereof (preferably sodium salt or palmitic acid ester), isoascorbic acid or metal salts thereof (preferably sodium salt), ethylenediamine tetraacetic acid or metal salts thereof (preferably calcium disodium salt or tetrasodium salt), cysteine, acetylcysteine, 2-mercaptobenzimidazole, 3(2)-t-butyl-4-hydroxyanisole, 2,6-di-t-butyl-4-methylphenol, tetrakis[3-(3',5'-di-t-butyl-4'-hydroxyphenyl)propionic acid]pentaerythritol, 3-mercapto-1,2-propanediol, tocopherol acetate, rutin, quercetin, hydroquinone, metal salts of hydroxymethanesulfinic acid (preferably sodium salts), metal metabisulfite salts (for example sodium salts), metal sulfite salts (preferably sodium salts) and metal thiosulfate salts (preferably sodium salts), and one species may be used or two or more species may be used in combination.

For the stabilizer, examples of the above-mentioned metals salts include sodium salts, potassium salts, calcium salts and magnesium salts. Examples of esters include palmitic acid esters, stearic acid esters and myristic acid esters.

There are no particular limitations on the proportion of the weight of the stabilizer as far as it does not exert an adverse effect on the properties of the pressure-sensitive adhesive layer. Preferable examples of the upper limit value of the proportion of the stabilizer based on the total weight of the pressure-sensitive adhesive layer (namely, the total weight of the pressure-sensitive adhesive layer in the adhesive preparation), in terms of the total content thereof, are such that a proportion in excess of 5 wt% results in the possibility of a decrease in the adhesiveness or other properties of the pressure-sensitive adhesive layer, while if the proportion is less than 0.0005 wt%, there is the possibility of adequate stabilizing effects being unable to be obtained. Thus, preferable examples of the upper limit value include 5 wt%, 3 wt%, 2 wt%, 1 wt%, 0.7 wt%, 0.5 wt% and 0.3 wt%, while preferable examples of the lower limit value are 0.0005 wt%, 0.001 wt%, 0.01 wt%, 0.02 wt%, 0.03 wt%, 0.05 wt%, 0.1 wt% and 0.2 wt%.

More specifically, the proportion of the total weight of the stabilizer based on the total weight of the pressure-sensitive adhesive layer (the total weight of the pressure-sensitive adhesive layer in the adhesive preparation) is preferably 0.0005 to 5 wt%, more preferably 0.001 to 3 wt%, more preferably 0.01 to 1 wt%, more preferably 0.01 to 0.91 wt%, more preferably 0.01 to 0.7 wt%, more preferably 0.02 to 0.7 wt%, more preferably 0.02 to 0.5 wt% and most preferably 0.03 to 0.3 wt%.
In the present invention, to suppress coloring of an adhesive preparation it is preferable that the production process of the adhesive preparation includes making the pressure-sensitive adhesive, the donepezil and the additives include in the pressure-sensitive adhesive layer. Although the additives may dissolve or disappear during storage of the adhesive preparation after the additives have demonstrated the effects of the present invention, this case is also included in the scope of the present invention.

In the present invention, "donepezil-related substances" refer to substances that are found in comparatively large amounts in pressure-sensitive adhesive layers containing donepezil, and are derived from donepezil (substances formed in association with donepezil; substances not found in pressure-sensitive adhesive layers not containing donepezil). Specific examples of donepezil-related substances include a related substance detected at a retention time of 12.8 minutes (hereinafter referred to as "related substance 1") and a related substance detected at a retention time of 3.9 minutes (hereinafter referred to as "related substance 2") in the case of analyzing the adhesive preparation of the present invention under the analysis conditions described in the subsequently described examples. As a result of blending two specific species of additives, the present invention demonstrates even greater usefulness with respect to inhibiting the formation of the specific related substance 1 and related substance 2, while also inhibiting the formation of other related substances, thereby making it possible to ultimately reduce the total amounts of related substances formed.

In the present invention, combining ascorbic acid, metal salts or esters thereof (hereinafter generically referred to as "ascorbic acid derivatives") with metal metabisulfite salt has the advantage of being able to reduce the amounts of ascorbic acid derivatives used while also being able to synergistically reduce the amounts of related substance 1 and related substance 2 formed as well as the total amounts of related substances formed.

In the adhesive preparation of the present invention, there are no particular limitations on the pressure-sensitive adhesive contained in the pressure-sensitive adhesive layer, and examples include acrylic pressure-sensitive adhesives; silicone rubber, polyisoprene rubber, polyisobutylene rubber, styrene-butadiene rubber, styrene-isoprene-styrene block copolymer rubber, styrene-butadiene-styrene block copolymer rubber and other rubber pressure-sensitive adhesives; silicone pressure-sensitive adhesives; and polyvinyl alcohol, polyvinyl alkyl ether, polyvinyl acetate and other vinyl polymer pressure-sensitive adhesives.

Since rubber pressure-sensitive adhesives often do not have highly reactive functional groups, the donepezil contained therein is comparatively stable and comparatively few related substances are formed. Examples of such rubber pressure-sensitive adhesives include polyisobutylene and styrene-diene-styrene block copolymers (such as styrene-butadiene-styrene block copolymer (SBS) and styrene-isoprene-styrene block copolymer (SIS)), and one species may be used or a mixture of two or more species thereof may be used.

Depending on the type and proportion of the copolymerized monomers, although acrylic pressure-sensitive adhesives have a comparatively high degree of freedom in terms of being able to control adhesive properties and the degree of drug solubility and the like, conversely their polymer chains may contain functional groups that are reactive with donepezil, and since residual monomers and polymerization initiators in the pressure-sensitive adhesive may also react with the donepezil, there is concern over a reduction in the effective content of donepezil. Thus, the present invention is carried out particularly advantageously in an adhesive preparation using an acrylic pressure-sensitive adhesive.

Examples of an acrylic pressure-sensitive adhesive in the present invention include acrylic pressure-sensitive adhesives containing a (meth)acrylic acid alkyl ester, and are preferably acrylic pressure-sensitive adhesives having a (meth)acrylic acid alkyl ester as a principal component (principal constituent unit) thereof. A copolymer of a (meth)acrylic acid alkyl ester (first monomer component) as the principal component with a vinyl monomer having functional groups capable of contributing to a crosslinking reaction (second monomer component), or a copolymer of these copolymerized with yet another monomer (third monomer component), is particularly preferable from the viewpoint of ease of crosslinking, pressure-sensitive adhesiveness with human skin, ability to manipulate drug dissolution and the like.

Preferable examples of the (meth)acrylic acid alkyl ester (first monomer component) include (meth)acrylic acid alkyl esters wherein the alkyl group is a linear, branched or cyclic alkyl group having 1 to 18 carbon atoms (such as methyl, ethyl, propyl, butyl, pentyl, hexyl, cyclohexyl, heptyl, octyl, 2-ethylhexyl, nonyl, decyl, undecyl, dodecyl or tridecyl), and (meth)acrylic acid alkyl esters wherein the alkyl group is a linear, branched or cyclic alkyl group having 4 to 18 carbon atoms (such as butyl, pentyl, hexyl, cyclohexyl, heptyl, octyl, 2-ethylhexyl, nonyl, decyl, undecyl, dodecyl or tridecyl). Moreover, since the use of a monomer component that lowers the glass transition temperature of the polymer is more preferable for imparting pressure-sensitive adhesiveness at normal temperatures, a (meth)acrylic acid alkyl ester wherein the alkyl group is a linear, branched or cyclic alkyl group having 4 to 8 carbon atoms (such as butyl, pentyl, hexyl, cyclohexyl, heptyl, octyl or 2-ethylhexyl, preferably butyl, 2-ethylhexyl or cyclohexyl and particularly preferably 2-ethylhexyl) is more preferable. More specifically, butyl acrylate, 2-ethylhexyl acrylate, 2-ethylhexyl methacrylate, cyclohexyl acrylate, cyclohexyl methacrylate or the like is preferable, and among these, 2-ethylhexyl acrylate is most preferable. One of these (meth)acrylic acid alkyl esters (first monomer component) may be used, or two or more may be used in combination.

In the vinyl monomer (second monomer component) having functional groups capable of contributing to the crosslinking reaction, examples of functional groups capable of contributing to the crosslinking reaction include hydroxyl groups, carboxyl groups and vinyl groups, and hydroxyl groups and carboxyl groups are preferable. Specific examples of this monomer (second monomer component) include hydroxyethyl (meth)acrylate esters, hydroxypropyl (meth)acrylate esters, (meth)acrylic acid, itaconic acid, maleic acid, maleic anhydride, mesaconic acid, citraconic acid and glutaconic acid and the like. Among these, acrylic acid, methacrylic acid and a hydroxyethyl acrylate ester (particularly 2-hydroxyethyl acrylate) are preferable from the viewpoint of availability, and acrylic acid is most preferable. One of these monomers (second monomer component) may be used, or two or more may be used in combination.

The above-mentioned other monomer (third monomer component) is used primarily to adjust the cohesiveness of the pressure-sensitive adhesive layer and to adjust the solubility or release properties of the donepezil and the like. Examples of this monomer (third monomer component) include vinyl acetate, vinyl propionate and other vinyl esters; methyl vinyl ether, ethyl vinyl ether and other vinyl ethers; N-vinyl-2-pyrrolidone, N-vinyl caprolactam and other vinyl amides; methoxyethyl (meth)acrylate ester, ethoxyethyl (meth)acrylate ester, tetrahydrofurfuryl (meth)acrylate ester and other alkoxy (meth)acrylate esters; hydroxypropyl (meth)acrylate, α-hydroxymethyl acrylate and other hydroxyl group-containing monomers (which do not provide crosslinking sites because they are used as the third monomer component); (meth)acrylamide, dimethyl (meth)acrylamide, N-butyl (meth)acrylamide, N-methylol (meth)acrylamide and other (meth)acrylic acid derivatives having amide groups; aminoethyl (meth)acrylate ester, dimethylaminoethyl (meth)acrylate ester, t-butylaminoethyl (meth)acrylate ester and other aminoalkyl (meth)acrylate esters; methoxyethylene glycol (meth)acrylate ester, methoxydiethylene glycol (meth)acrylate ester, methoxypolyethylene glycol (meth)acrylate ester, methoxypolypropylene glycol (meth)acrylate ester and other alkoxyalkylene glycol (meth)acrylate esters; (meth)acrylonitrile; styrene sulfonic acid, allyl sulfonic acid, sulfopropyl (meth)acrylate, (meth)acryloyl oxynaphthalene sulfonic acid, acrylamide methyl sulfonic acid and other monomers having sulfonic acid; and vinyl piperidone, vinyl pyrimidine, vinyl piperazine, vinyl pyrrole, vinyl imidazole, vinyl oxazole, vinyl morpholine and other vinyl group-containing monomers. Among these, a vinyl ester or vinyl amide is preferable, and vinyl acetate is preferable as a vinyl ester, while N-vinyl-2-pyrrolidone is preferable as a vinyl amide. One of these monomers (third monomer component) may be used or two or more may be used in combination.

When the acrylic pressure-sensitive adhesive is a copolymer of a (meth)acrylic acid alkyl ester (first monomer component) and a vinyl monomer having functional groups capable of contributing to a crosslinking reaction (second monomer component), the (meth)acrylic acid alkyl ester and the vinyl monomer having functional groups capable of contributing to a crosslinking reaction are blended and copolymerized preferably at a weight ratio of 99 to 85 parts of (meth)acrylic acid alkyl ester per 1 to 15 parts of vinyl monomer having functional groups capable of contributing to a crosslinking reaction, and more preferably at a weight ratio of 99 to 90 parts per 1 to 10 parts.

In addition, when the acrylic pressure-sensitive adhesive is a copolymer of a (meth)acrylic acid alkyl ester (first monomer component), a vinyl monomer having functional groups capable of contributing to a crosslinking reaction (second monomer component) and another monomer (third monomer component), the (meth)acrylic acid alkyl ester, vinyl monomer having functional groups capable of contributing to a crosslinking reaction and other monomer are blended and copolymerized preferably at a weight ratio of 40 to 94 parts of (meth)acrylic acid alkyl ester per 1 to 15 parts of vinyl monomer having functional groups capable of contributing to a crosslinking reaction and 5 to 50 parts of other monomer, and more preferably at a weight ratio of 50 to 89 parts per 1 to 10 parts and 10 to 40 parts, respectively.

Although there are no particular limitations on the polymerization reaction as far as it is carried out with a known method, as an example thereof, a polymerization initiator (such as benzoyl peroxide, azobisisobutyronitrile or the like) is added to the above-mentioned monomers followed by reacting for 5 to 48 hours at 50 to 70°C in a solvent (such as ethyl acetate).

Particularly preferable examples of acrylic pressure-sensitive adhesives in the present invention include 2-ethylhexyl acrylate ester/acrylic acid/N-vinyl-2-pyrrolidone copolymer, 2-ethylhexyl acrylate ester/2-hydroxyethyl acrylate ester/vinyl acetate copolymer and 2-ethylhexyl acrylate ester/acrylic acid copolymer and the like, while a more preferable example is 2-ethylhexyl acrylate ester/acrylic acid/N-vinyl-2-pyrrolidone copolymer.

In addition, although varying according to the copolymer composition, the glass transition temperature of the acrylic pressure-sensitive adhesive in the present invention is normally preferably -100 to -10°C and more preferably -90 to -20°C from the viewpoint of adhesiveness of the adhesive preparation.

In the adhesive preparation of the present invention, a liquid component can be included in the pressure-sensitive adhesive layer from the viewpoint of imparting softness to the pressure-sensitive adhesive layer and reducing pain and skin irritation caused by skin adhesiveness when the adhesive preparation is peeled off the skin. An organic liquid component is preferable from the viewpoint of compatibility with the pressure-sensitive adhesive layer. In an adhesive preparation of the present invention containing an organic liquid component, there is the possibility of the stability of the donepezil decreasing due to a chemical reaction with the donepezil and the like depending on the type of organic liquid component. Thus, the present invention is carried out particularly advantageously in the case of an adhesive preparation containing an organic liquid component in terms of being able to efficiently inhibit this decrease in stability.

Although the organic liquid component can be used without any particular limitations as far as it is a liquid at room temperature, exhibits a plasticizing action and is compatible with the pressure-sensitive adhesive polymers composing the pressure-sensitive adhesive, that which improves the percutaneous absorbability and storage stability of donepezil is preferable. In addition, the organic liquid component can also be blended for the purpose of further enhancing the solubility of donepezil in the pressure-sensitive adhesive and the like. Examples of such organic liquid components include fatty acid alkyl esters (such as esters of lower monovalent alcohols having 1 to 4 carbon atoms and saturated or unsaturated fatty acids having 12 to 16 carbon atoms); saturated or unsaturated fatty acids having 8 to 10 carbon atoms (such as caprylic acid (octanoic acid, C8), pelargonic acid (nonanoic acid, C9), capric acid (decanoic acid, C10) or lauric acid (C12)); ethylene glycol, diethylene glycol, triethylene glycol, polyethylene glycol, propylene glycol, polypropylene glycol and other glycols; olive oil, castor oil, squalene, lanoline and other oils and fats; ethyl acetate, ethyl alcohol, dimethyl decyl sulfoxide, decyl methyl sulfoxide, dimethyl sulfoxide, dimethyl formamide, dimethyl acetamide, dimethyl lauryl amide, dodecyl pyrrolidone, isosorbitol, oleyl alcohol and other organic solvents; liquid surfactants; diisopropyl adipate, phthalic acid esters, diethyl sebacate and other plasticizers; and liquid paraffin and other hydrocarbons. In addition, other examples include ethoxylated stearyl alcohol, glycerin esters (those liquid at room temperature), isotridecyl myristate, N-methylpyrrolidone, ethyl oleate, oleic acid, diisopropyl adipate, octyl palmitate, 1,3-propanediol and glycerin. Among these, a fatty acid alkyl ester, saturated fatty acid, hydrocarbon or organic solvent is preferable from the viewpoint of stability of the preparation and the like, and a fatty acid alkyl ester is more preferable. One of these liquid organic components may be used alone or two or more may be used in combination.

In addition, in the case of using an acrylic pressure-sensitive adhesive for the pressure-sensitive adhesive, the organic liquid component is preferably a fatty acid alkyl ester from the viewpoint of compatibility and the like with the acrylic pressure-sensitive adhesive, and is more preferably an ester of a lower monovalent alcohol having 1 to 4 carbon atoms and a saturated or unsaturated fatty acid having 12 to 16 carbon atoms. Here, the saturated or unsaturated fatty acid having 12 to 16 carbon atoms is preferably a saturated fatty acid, while the lower monovalent alcohol having 1 to 4 carbon atoms may be either linear or branched. Preferable examples of fatty acids having 12 to 16 carbon atoms include lauric acid (C12), myristic acid (C14) and palmitic acid (C16) and the like, while preferable examples of lower monovalent alcohols having 1 to 4 carbon atoms include isopropyl alcohol, ethyl alcohol, methyl alcohol and propyl alcohol and the like. Specific examples of particularly preferable fatty acid alkyl esters include isopropyl myristate, ethyl laurate and isopropyl palmitate and the like.

Furthermore, in the case of using a fatty acid alkyl ester, a fatty acid having 8 to 10 carbon atoms and/or glycerin may be used in combination with the fatty acid alkyl ester from the viewpoint of improving the percutaneous absorbability of the donepezil.

The content of the organic liquid component blended in the present invention is preferably 10 to 160 parts by weight and more preferably 40 to 150 parts by weight based on 100 parts by weight of the pressure-sensitive adhesive. If the content is less than 10 parts by weight, favorable softness or the effect of reducing skin irritation may not be adequately obtained due to inadequate plasticization of the pressure-sensitive adhesive layer, while if the content exceeds 160 parts by weight, the organic liquid component may not be retained in the pressure-sensitive adhesive even by the cohesive force of the pressure-sensitive adhesive, thereby resulting in the adhesive force being weakened due to blooming on the surface of the pressure-sensitive adhesive layer, and increasing the possibility of the preparation detaching from the skin surface during use.

In the adhesive preparation of the present invention, the pressure-sensitive adhesive layer can be crosslinked by for example, a known chemical crosslinking process (crosslinking process using a crosslinking agent and the like) or physical crosslinking process (crosslinking process by exposing to ultraviolet rays or gamma (γ) rays or other electron rays and the like) as previously described, this crosslinking process may be that commonly used in the technical field. In the adhesive preparation of the present invention, the stability of the donepezil may decease (namely, the amounts of related substances formed may increase) during manufacture or storage of the preparation depending on the chemical or physical crosslinking process used. Thus, the present invention is carried out particularly advantageously in an adhesive preparation in which the pressure-sensitive adhesive layer has been crosslinked. Furthermore, a chemical crosslinking process using a crosslinking agent is preferable from the viewpoint of being less likely to adversely affect the donepezil.

In the case of carrying out a chemical crosslinking process using a crosslinking agent, there are no particular limitations on the crosslinking agent so far as crosslink formation by such a crosslinking agent is not inhibited in the presence of donepezil, and examples include peroxides (such as benzoyl peroxide (BPO) and the like), metal oxides (such as magnesium metasilicate aluminate and the like), polyfunctional isocyanate compounds, organic metal compounds (such as zirconium alaninate, zinc alaninate , zinc acetate, glycine ammonium zinc or titanium compounds), metal alcoholates (such as tetraethyl titanate, tetraisopropyl titanate, aluminum isopropylate or aluminum sec-butyrate) and metal chelate compounds (such as dipropoxy bis(acetylacetonate) titanium, tetraoctylene glycol titanium, aluminum isopropylate, ethylacetoacetate aluminum diisopropylate, aluminum tris(ethylacetoacetate) or aluminum tris(acetylacetonate)). Among these, a peroxide, metal oxide, organic metal compound, metal alcoholate or metal chelate compound is preferable, and a metal alcoholate or metal chelate compound is more preferable from the viewpoint of efficiently forming crosslinks in the presence of donepezil, while a metal chelate compound is most preferable from the viewpoint of easily obtaining crosslinked structures with a suitable crosslinking density. In addition, among the metal chelate compounds, ethylacetoacetate aluminum diisopropylate is particularly preferable. One of these crosslinking agents may be used or two or more may be used in combination.

Although varying according to the type of crosslinking agent and pressure-sensitive adhesive, the content of the crosslinking agent blended is typically 0.1 to 0.6 parts by weight and preferably 0.15 to 0.5 parts by weight based on 100 parts by weight of the pressure-sensitive adhesive. If the content is less than 0.1 parts by weight, the crosslinking sites are too few to impart adequate cohesiveness to the pressure-sensitive adhesive layer, resulting in the risk of adhesive residue and strong skin irritation due to cohesive failure during peeling, while if the content exceeds 0.6 parts by weight, although cohesiveness is large, there are cases in which adequate adhesiveness may be unable to be obtained. In addition, there is also the risk of skin irritation caused by residual unreacted crosslinking agent.

Chemical crosslinking treatment can be carried out by, for example, adding the crosslinking agent followed by going through a step consisting of heating and storing at or above the crosslinking reaction temperature, or in other words a curing step. The heating temperature at this time is suitably selected according to the type of crosslinking agent, it is preferably 60 to 90°C and more preferably 60 to 80°C. The heating time is preferably 12 to 96 hours and more preferably 24 to 72 hours.

In the adhesive preparation of the present invention, a metal chloride can be contained together with donepezil in the crosslinked pressure-sensitive adhesive layer. Containing a metal chloride in the pressure-sensitive adhesive layer reduces the decrease in cohesiveness of the pressure-sensitive adhesive layer and the adhesive preparation is attached to human skin, and makes it less likely for cohesive failure to occur when the pressure-sensitive adhesive layer is peeled off.

There are no particular limitations on the metal chloride, and examples thereof include a chloride of an alkaline metal such as sodium or potassium; a chloride of an alkaline earth metal such as calcium or magnesium; aluminum chloride, stannous chloride and ferric chloride. From the viewpoint of superior stability and ability to inhibit decreases in cohesiveness of the pressure-sensitive adhesive layer, sodium chloride, calcium chloride, aluminum chloride, stannous chloride or ferric chloride is preferable, sodium chloride or calcium chloride is more preferable, and sodium chloride is particularly preferable. Any of these may be used alone or two or more may be used in combination. The content of the metal chloride blended is preferably 0.1 to 20 parts by weight, more preferably 1 to 15 parts by weight and most preferably 3 to 10 parts by weight based on 100 parts by weight of the pressure-sensitive adhesive. If the content is less than 0.1 parts by weight, the effect of inhibiting decreases in cohesiveness of the pressure-sensitive adhesive layer may be inadequate, while conversely if the content exceeds 20 parts by weight, although the inhibitory effect is demonstrated, the appearance of the preparation may be impaired due to the metal chloride not being uniformly dispersed in the pressure-sensitive adhesive (pressure-sensitive adhesive polymer).

In the present invention, the metal chloride may be that produced by neutralizing donepezil hydrochloride with an inorganic base containing a metal in the process of forming the pressure-sensitive adhesive layer. Examples of such inorganic bases containing metals include sodium hydroxide, potassium hydroxide, calcium hydroxide, magnesium hydroxide, sodium hydrogencarbonate, potassium hydrogencarbonate, sodium carbonate, potassium carbonate and other inorganic bases of alkaline metals or alkaline earth metals, a hydroxide of an alkaline metal or alkaline earth metal is preferable from the viewpoint of less likelihood of the formation of by-products, sodium hydroxide, calcium hydroxide and magnesium hydroxide are more preferable, and sodium hydroxide is particularly preferable.

In the adhesive preparation of the present invention, the thickness of the pressure-sensitive adhesive layer is preferably 20 to 300 µm, more preferably 30 to 300 µm and most preferably 50 to 300 µm. If the thickness of the pressure-sensitive adhesive layer is less than 20 µm, there is the risk of it being difficult to obtain adequate adhesive force or contain an effective content of donepezil, while if the thickness exceeds 300 µm, there is the risk of coating being difficult.

The adhesive preparation of the present invention has a support and a pressure-sensitive adhesive layer, and is preferably provided with a release liner. Namely, the adhesive preparation of the present invention has a structure wherein the pressure-sensitive adhesive layer described above is laminated on at least one side of the support, and the adhesive surface of the pressure-sensitive adhesive layer (the surface opposite the side on which the pressure-sensitive adhesive layer is laminated on the support) is preferably protected by being covered with a release liner until immediately before use. In addition, the adhesive preparation of the present invention may also be in the form of a roll without using a release liner by coating a silicone, fluorine or wax backing agent and the like onto the support.

Although there are no particular limitations on the support, that in which the proportion of donepezil in the pressure-sensitive adhesive layer does not decrease as a result of being lost from the back of the support by passing there through (namely, a material impermeable to donepezil) is preferable. And as will be described below, in the case of an aspect in which an organic liquid component is contained in the pressure-sensitive adhesive layer, a support in which the proportions of donepezil and organic liquid component do not decrease as a result of being lost from the back of the support by passing there through (namely, a material impermeable to the organic liquid component and the donepezil) is preferable.

Specific examples include polyester (such as polyethylene terephthalate (PET)), nylon, polyvinyl chloride, polyethylene, polypropylene, ethylene-vinyl acetate copolymer, polytetrafluoroethylene, ionomer resins and other single films, metal foil, and laminate films obtained by laminating two or more such films. Among these, the support is preferably that in which a laminate film is obtained by laminating a nonporous film consisting of one of the aforementioned materials with a porous film as described below in order to improve the adhesiveness (anchoring properties) of the support with the pressure-sensitive adhesive layer, and the pressure-sensitive adhesive layer is formed on the side of the porous film.

There are no particular limitations on the porous film as far as it improves anchoring properties with the pressure-sensitive adhesive layer, and examples include paper, woven fabrics, nonwoven fabrics (such as polyester (including polyethylene terephthalate (PET)) nonwoven fabric)), and films obtained by mechanical perforation of the above-mentioned films (such as polyester, nylon, Saran (product name), polyethylene, polypropylene, ethylene-vinyl acetate copolymer, polyvinyl chloride, ethylene-ethyl acrylate copolymer, polytetrafluoroethylene, metal foil, polyethylene terephthalate and other single films and laminate films obtained by laminating one or two or more such films), while paper, woven fabrics and nonwoven fabrics (such as polyester nonwoven fabric or polyethylene terephthalate nonwoven fabric) are preferable from the viewpoint of flexibility of the support. In consideration of improvement of anchoring properties and flexibility of the pressure-sensitive adhesive layer, the thickness of the porous film is normally 10 to 500 µm, and in the case of a thin adhesive preparation such as a plaster or adhesive tape, the thickness is normally about 1 to 200 µm. In the case of woven fabrics or nonwoven fabrics, the basis weight is preferably 5 to 30 g/m² in terms of improving anchoring properties.

There are no particular limitations on the thickness of the support for the adhesive preparation of the present invention, and is preferably 2 to 200 µm and more preferably 10 to 50 µm. If the thickness of the support is less than 2 µm, handling ease such as self-supporting properties tend to decrease, while if the thickness exceeds 200 µm, the support may feel unpleasant (stiff) and compliance with the skin tends to decrease.

There are no particular limitations on the release liner, and a known release liner can be used. Specific examples of the release liner include a release liner in which a release agent layer composed of a release agent is formed on the surface of a release liner base, a plastic film which itself has good release properties, and a release liner of a configuration in which a release layer, consisting of the plastic film material with good release properties, is formed on the surface of a release liner base. The release surface of the release liner may be on only one side of the base or on both sides.

There are no particular limitations on the release agent in this release liner, examples of which include long-chain alkyl group-containing polymers, silicone polymers (silicon release agents), fluorine polymers (fluorine release agents) and other release agents. Examples of the base for the release liner include polyethylene terephthalate (PET) film, polyimide film, polypropylene film, polyethylene film, polycarbonate film, polyester (other than PET) film or other plastic film; metal-deposited plastic films obtained by depositing a metal on one of these films; Japanese paper, regular paper, kraft paper, glassine paper, fine paper other paper; nonwoven fabric, fabric or other fibrous material; and metal foil.

In addition, examples of plastic films that themselves have good release properties include polyethylene (such a low-density polyethylene or linear low-density polyethylene), polypropylene, ethylene-propylene copolymer and other ethylene-α-olefin copolymers (block copolymers or random copolymers) as well as polyolefin films formed from polyolefin resins consisting of mixtures thereof, and Teflon (registered trademark) films.

Furthermore, the release layer formed on the surface of the above-mentioned release liner base can be formed by laminating or coating the material of the above-mentioned plastic film with good release properties on the above-mentioned release liner base.

There are no particular limitations on the thickness (total thickness) of the release liner, and is normally 200 µm or less and preferably 25 to 100 µm.

In the present invention, although there are no particular limitations on the method for preparing the adhesive preparation, in one embodiment thereof, the adhesive preparation of the present invention is manufactured by film-forming of a mixture containing at least a pressure-sensitive adhesive, donepezil and the above-mentioned two species of additives to form the pressure-sensitive adhesive layer containing donepezil. Namely, the present invention also relates to a method for manufactured an adhesive preparation containing donepezil, which comprises forming a pressure-sensitive adhesive layer containing donepezil on at least one side of a support by film-forming of a mixture containing a pressure-sensitive adhesive, donepezil and the above-mentioned stabilizer. In addition, the present invention relates to a method for stabilizing donepezil in an adhesive preparation, which comprises including the above-mentioned two species of additives together with donepezil in the presence of a pressure-sensitive adhesive. Moreover, the present invention also relates to a method for suppressing coloring of a donepezil-containing adhesive preparation, which comprises including the above-mentioned two species of additives together with the donepezil in the presence of a pressure-sensitive adhesive.

In this embodiment, since the above-mentioned two species of additives are blended with the donepezil in the mixture for forming the pressure-sensitive adhesive layer, in addition to coloring of the donepezil-containing adhesive preparation being suppressed, the donepezil is stabilized from the time the mixture is prepared for forming the pressure-sensitive adhesive layer, and the formation of the related substances can thus be adequately inhibited.

A specific example of a method consists of dissolving or dispersing a pressure-sensitive adhesive, donepezil, the above-mentioned two species of additives, and a stabilizer and the like in a solvent and mixing, coating the resulting solution or dispersion on at least one side of a support, and drying and depositing as a film to form the pressure-sensitive adhesive layer on the surface of the support followed by providing a release liner. Alternatively, for example, the above-mentioned solution or dispersion can be applied to at least one side of a protective release liner and then dried and deposited as a film to form the pressure-sensitive adhesive layer on the surface of the release liner, followed by adhering the support to the pressure-sensitive adhesive layer to prepare the adhesive preparation.

Examples of the solvent for dissolving and/or dispersing the pressure-sensitive adhesive and the like include ethyl acetate, toluene, hexane, 2-propanol, methanol, ethanol and water. In addition, these can also be used to adjust the viscosity after adding the crosslinking agent.

Furthermore, in the case the pressure-sensitive adhesive layer is to be crosslinked, the crosslinking agent is preferably added to the above-mentioned solution or dispersion as a chemical crosslinking treatment. In addition, in the case the pressure-sensitive adhesive layer is to be crosslinked, the pressure-sensitive adhesive layer is preferably stored (aging treatment (aging step)) to promote crosslinking in the pressure-sensitive adhesive layer after film formation. The aging treatment (aging step) is normally carried out after the solution or dispersion has been coated and dried to form a film (after formation of the pressure-sensitive adhesive layer) by allowing the resulting pressure-sensitive adhesive layer to stand for about 12 to 96 hours (preferably about 24 to 72 hours) while heating at 60 to 90°C (preferably 60 to 80°C). During this time, the donepezil can be stabilized due to the presence of the above-mentioned two species of additives, thereby inhibiting the formation of related substances.

There are no particular limitations on the form of the adhesive preparation of the present invention, and may be in the form of a tape, sheet, matrix, reserver, controlled release film and the like. The stabilizing effect of the present invention is advantageous for tapes and sheets because these are susceptible to the effects of the environment and particularly oxygen.

Although varying according to the type and the content of pressure-sensitive adhesive and organic liquid component used as well as patient age, body weight, symptoms and the like, the dosage of the adhesive preparation of the present invention is preferably such that, in the case of an adult, an adhesive preparation containing 2 to 150 mg of donepezil or donepezil hydrochloride is normally administered to a 5 to 120 cm² patch of skin for about 1 to 7 days.

### [Examples]

The following provides a more detailed explanation of the present invention through examples thereof. Furthermore, unless otherwise specified, "parts" in the description refers to "parts by weight".

### Examples 1 to 4 and Comparative Examples 1 and 2

In an inert gas atmosphere, 75 parts of 2-ethylhexyl acrylate, 22 parts of N-vinyl-2-pyrrolidone, 3 parts of acrylic acid and 0.2 parts of azobisisobutyronitrile were solution-polymerized in ethyl acetate at 60°C to obtain an ethyl acetate solution of pressure-sensitive adhesive A (pressure-sensitive adhesive solid content: 28%).

Next, coating solutions, which contain the additives shown in Table 2 and Table 3 below, were obtained by mixing the components shown in Table 1 below and adjusting the viscosity with ethyl acetate. These were coated onto polyethylene terephthalate (PET) release liners to a dried thickness of 60 µm, dried and laminated onto PET supports to obtain adhesive preparations followed by storing for 48 hours at 70°C to obtain the stored adhesive preparations of Examples 1 to 4 and Comparative Examples 1 and 2.

**[Table 1]**

| | |
|---|---|
| Pressure-sensitive adhesive A | 40 parts |
| Isopropyl myristate | 50 parts |
| Donepezil hydrochloride | 8.3 parts |
| Sodium hydroxide | 0.8 parts |
| Ethyl acetoacetate aluminum diisopropylate | 0.2 parts |
| Additives | As indicated in Table 2 or Table 3 |

### Reference Example 1

The stored adhesive preparation (placebo preparation) of Reference Example 1 was obtained by treating in the same manner as Example 1 with the exception of not blending the donepezil hydrochloride and the two species of additives.

### Reference Example 2

The stored adhesive preparation (control preparation) of Reference Example 2 was obtained by treating in the same manner as Example 1 with the exception of not blending the two species of additives.

### (Measurement of Coloring)

Color values (L*,a*,b*) represented by the CIE1976 (L*a*b*) color system (L-star, a-star, b-star color system, JIS Z 8729) were measured, and color differences (ΔE*ab1) between the color values (L*1,a*1,b*1) of the adhesive preparations of Examples 1 to 4 and Comparative Examples 1 and 2 along with the adhesive preparation of Reference Example 2 (control preparation) and the color value (L*s,a*s,b*s) of the adhesive preparation of Reference Example 1 (placebo preparation) as reference point were calculated according to formula 1 below. The results are shown in Table 2.

ΔAE*ab1 = [(L*1-L*s)² + (a*1-a*s)² + (b*1-b*s)²]^{1/2} (Formula 1)

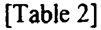

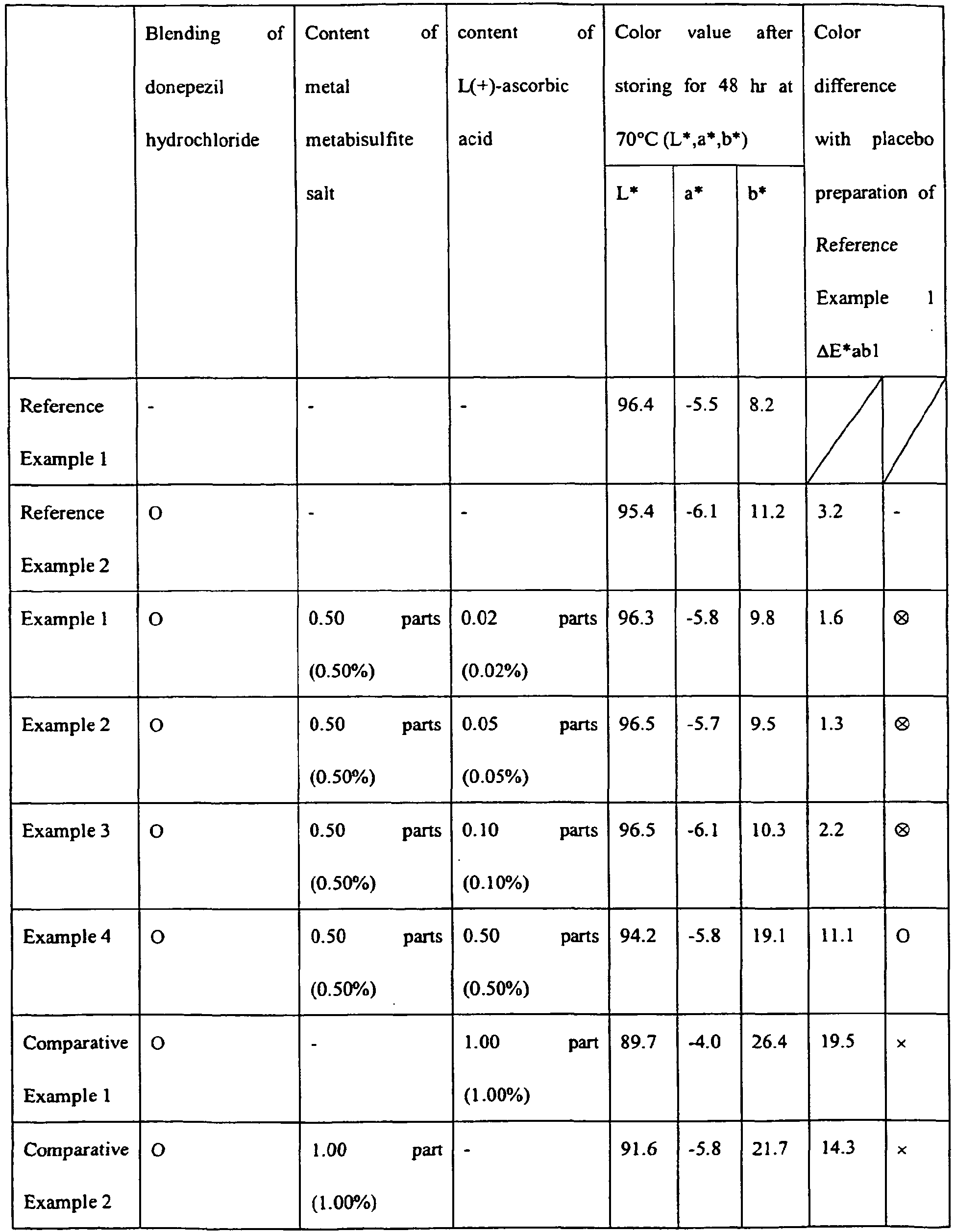

### (Measurement of Proportion of Related Substances)

The adhesive preparations of the Examples 1 to 4, Comparative Examples 1 and 2 and Reference Example 2 were extracted with methanol, and the extracts were analyzed by HPLC under the following conditions.

### (HPLC conditions)

HPLC column: Inertsil (registered trademark) ODS-2 (4.6 mm I.D. x 15 cm, 5 µm) GL Science
Column temperature: 35°C
Mobile phase: Sodium 1-decansulfonate aqueous solution/acetonitrile/70% perchloric acid = 650/350/1 (volume ratio)
Sodium 1-decansulfonate concentration: 10 mM based on total mobile phase
Flow rate: 1.4 mL/min
Detection: UV (271 nm)
Retention times: Donepezil =11.0 min., related substance 1 = 12.8 min., related substance 2= 3.9 min.

The area ratios of the areas of the HPLC peaks for donepezil related substances 1 and 2 and total related substances to the area of the HPLC peak for donepezil in the pressure-sensitive adhesive layers of the stored adhesive preparations of Examples 1 to 4 and Comparative Examples 1 and 2, and Reference Example 2 are shown in Table 3 as the proportion of donepezil-related substance 1, the proportion of donepezil-related substance 2 and the proportion of all donepezil-related substances, respectively.

**[Table 3]**

| | Metal metabisulfite salt | L(+)-ascorbic acid | Proportion of related substance 1 | Proportion of related substance 2 | Proportion of total related substances |
|---|---|---|---|---|---|
| Ref. Ex. 2 | - | - | 1.2% | 0.2% | 1.9% |
| Example 1 | 0.50 parts (0.50%) | 0.02 parts (0.02%) | nd | 0.0% | 0.4% |
| Example 2 | 0.50 parts (0.50%) | 0.05 parts (0.05%) | nd | 0.0% | 0.2% |
| Example 3 | 0.50 parts (0.50%) | 0.10 parts (0.10%) | nd | 0.0% | 0.2% |
| Example 4 | 0.50 parts (0.50%) | 0.50 parts (0.50%) | nd | 0.0% | 0.2% |
| Comp. Ex. 1 | - | 1.00 part (1.00%) | 0.5% | 0.0% | 0.6% |
| Comp. Ex. 2 | 1.00 part (1.00%) | part - | nd | 1.0% | 3.6% |
| Ref. Ex. 3 | Arithmetic mean of Comp. Ex. 1 and Comp. Ex. 2 | | 0.25% | 0.5% | 2.1% |

| | | | | | |
|---|---|---|---|---|---|
| Note: nd in the table means "not detected". | | | | | |

As is clear from Table 2, the adhesive preparations of Examples 1 to 4 containing ascorbic acid derivatives and metal metabisulfite salt demonstrated smaller values for color difference (ΔE*ab1) with the placebo preparation of Reference Example 1 than those of Comparative Examples 1 and 2. On the basis thereof, it is recognized that coloring of the donepezil-containing adhesive preparations was suppressed and a naturally colored appearance was realized by blending these two species of additives.
Moreover, as is clear from a comparison of the comparative examples, reference examples and examples, the adhesive preparations of Examples 1 to 4 unexpectedly demonstrated smaller values for color difference (ΔE*ab1) with the placebo preparation of Reference Example 1 than the control preparation of Reference Example 2, despite containing ascorbic acid derivatives and metal metabisulfite salt. On the basis thereof, the adhesive preparations of Examples 1 to 4 were confirmed to realize natural coloring as donepezil-containing adhesive preparations.
In particular, as a result of blending 0.04 to 0.2 parts by weight of ascorbic acid derivatives (ascorbic acid, metal salts or esters thereof) to 1.0 part by weight of metal metabisulfite salt, the adhesive preparations of Examples 1 to 3 demonstrated extremely small values for the color difference (ΔE*ab1) with the placebo preparation of Reference Example 1, thereby confirming further suppression of coloring of donepezil-containing adhesive preparations and the realization of an extremely naturally colored appearance.

In addition, as is clear from Table 3, the adhesive preparations of Examples 1 to 4 blending ascorbic acid derivatives and metal metabisulfite salt were confirmed to reduce the amounts of related substance 1 formed, related substance 2 formed and total related substances formed in comparison with Comparative Example 1, Comparative Example 2 and Reference Example 2. Moreover, the adhesive preparations of Examples 1 to 4 were also confirmed to synergistically reduce the amounts of related substance 1 formed, related substance 2 formed and total related substances formed based on a comparison with Reference Example 3 (arithmetic mean of Comparative Example 1 and Comparative Example 2).

According to the present invention, since coloring of a donepezil-containing adhesive preparation can be suppressed to realize a naturally colored appearance, and since the amounts of specific donepezil-related substances formed in the preparation can be reliably reduced, the present invention can be widely and effectively used as an anti-Alzheimer's dementia drug, anti-cerebrovascular dementia drug, prevention of migraine headaches and other pharmaceutical applications by parenteral administration, and particularly by percutaneous administration.

## Claims

1. A method for suppressing coloring of an adhesive preparation having a support and a pressure-sensitive adhesive layer, the pressure-sensitive adhesive layer containing a pressure-sensitive adhesive and donepezil, the method comprising:
a step of including ascorbic acid, a metal salt or an ester thereof and metal metabisulfite salt in the pressure-sensitive adhesive layer.

2. The method according to claim 1, which comprises:
a step of preparing a mixture comprising the pressure-sensitive adhesive, the donepezil, the ascorbic acid, a metal salt or an ester thereof, and the metal metabisulfite salt; and
a step of film-forming the pressure-sensitive adhesive layer by applying the mixture to a surface of the support.

3. The method according to claim 1 or 2, comprising:
blending 0.02 to 1.5 parts by weight of the ascorbic acid, a metal salt or an ester thereof to 1.0 part by weight of the metal metabisulfite salt.

4. A method for suppressing coloring of an adhesive preparation having a support and a pressure-sensitive adhesive layer, the pressure-sensitive adhesive layer containing a pressure-sensitive adhesive and donepezil, and reducing the amounts of donepezil-related substances formed, the method comprising:
a step of including ascorbic acid, a metal salt or an ester thereof and metal metabisulfite salt in the pressure-sensitive adhesive layer.

5. The method according to claim 4, which comprises:
a step of preparing a mixture comprising the pressure-sensitive adhesive, the donepezil, the ascorbic acid, a metals salt or an ester thereof, and the metal metabisulfite salt; and
a step of film-forming the pressure-sensitive adhesive layer by applying the mixture to a surface of the support.

6. The method according to claim 4 or 5, comprising:
blending 0.02 to 1.5 parts by weight of the ascorbic acid, a metal salt or an ester thereof to 1.0 part by weight of the metal metabisulfite salt.

## Patentansprüche

1. Verfahren zum Unterdrücken der Verfärbung einer klebenden Zubereitung, die einen Träger und eine druckempfindliche Klebemittelschicht umfasst, wobei die druckempfindliche Klebemittelschicht ein druckempfindliches Klebemittel und Donepezil umfasst, wobei das Verfahren umfasst:
einen Schritt zum Inkludieren von Ascorbinsäure, einem Metallsalz oder einem Ester hiervon und Metall-Metabisulfitsalz in die druckempfindliche Klebemittelschicht.

2. Verfahren gemäß Anspruch 1, welches umfasst:
einen Schritt zum Zubereiten einer Mischung, die ein druckempfindliches Klebemittel, das Donepezil, die Ascorbinsäure, ein Metallsalz oder einen Ester hiervon und das Metall-Metabisulfitsalz umfasst; und
einen Schritt zum Filmbilden der druckempfindlichen Klebemittelschicht durch Auftragen der Mischung auf eine Oberfläche des Trägers.

3. Verfahren gemäß Anspruch 1 oder 2, umfassend:
Vermischen von 0,02 bis 1,5 Gew.-Teile von Ascorbinsäure, einem Metallsalz oder einem Ester hiervon mit 1,0 Gew.-Teilen des Metall-Metabisulfitsalzes.

4. Verfahren zum Unterdrücken der Verfärbung einer klebenden Zubereitung, die einen Träger und eine druckempfindliche Klebemittelschicht umfasst, wobei die druckempfindliche Klebemittelschicht ein druckempfindliches Klebemittel und Donepezil enthält, und zum Verringern der Mengen von mit Donepezil in Zusammenhang stehenden gebildeten Substanzen, wobei das Verfahren umfasst:
einen Schritt zum Inkludieren von Ascorbinsäure, einem Metallsalz oder einem Ester hiervon und von Metall-Metabisulfitsalz in die druckempfindliche Klebemittelschicht.

5. Verfahren gemäß Anspruch 4, welches umfasst:
einen Schritt zum Zubereiten einer Mischung, die das druckempfindliche Klebemittel, das Donepezil, die Ascorbinsäure, ein Metallsalz oder einen Ester hiervon und das Metall-Metabisulfitsalz umfasst; und
einen Schritt zum Filmbilden der druckempfindlichen Klebemittelschicht durch Auftragen der Mischung auf eine Oberfläche des Trägers.

6. Verfahren gemäß Anspruch 4 oder 5, umfassend:
Vermischen von 0,02 bis 1,5 Gew.-Teilen von Ascorbinsäure, einem Metallsalz oder einem Ester hiervon mit 1,0 Gew.-Teilen des Metall-Metabisulfitsalzes.

## Revendications

1. Procédé pour supprimer la coloration d'une préparation adhésive ayant un support et une couche adhésive sensible à la pression, la couche adhésive sensible à la pression contenant un adhésif sensible à la pression et du donépézil, le procédé comprenant :
une étape d'inclusion d'acide ascorbique, d'un sel de métal ou d'un ester de celui-ci et d'un sel de métabisulfite de métal dans la couche adhésive sensible à la pression.

2. Procédé selon la revendication 1, qui comprend :
une étape de préparation d'un mélange comprenant l'adhésif sensible à la pression, le donépézil, l'acide ascorbique, un sel de métal ou un ester de celui-ci, et le sel de métabisulfite de métal ; et
une étape de formation de film de la couche adhésive sensible à la pression en appliquant le mélange à une surface du support.

3. Procédé selon la revendication 1 ou 2, comprenant :
le mélange de 0,02 à 1,5 partie en poids de l'acide ascorbique, d'un sel de métal ou d'un ester de celui-ci à 1,0 partie par poids du sel de métabisulfite de métal.

4. Procédé pour supprimer la coloration d'une préparation adhésive ayant un support et une couche adhésive sensible à la pression, la couche adhésive sensible à la pression contenant un adhésif sensible à la pression et du donépézil, et pour réduire les quantités de substances formées liées au donépézil, le procédé comprenant :
une étape d'inclusion d'acide ascorbique, d'un sel de métal ou d'un ester de celui-ci et d'un sel de métabisulfite de métal dans la couche adhésive sensible à la pression.

5. Procédé selon la revendication 4, qui comprend :
une étape de préparation d'un mélange comprenant l'adhésif sensible à la pression, le donépézil, l'acide ascorbique, un sel de métaux ou un ester de celui-ci, et le sel de métabisulfite de métal ; et
une étape de formation de film de la couche adhésive sensible à la pression en appliquant le mélange à une surface du support.

6. Procédé selon la revendication 4 ou 5, comprenant :
le mélange de 0,02 à 1,5 partie en poids de l'acide ascorbique, d'un sel de métal ou d'un ester de celui-ci à 1,0 partie en poids du sel de métabisulfite de métal.
